# EUROPEAN PATENT SPECIFICATION

(11) **EP 1 126 279 B1**
(45) Date of publication and mention of the grant of the patent: **03.09.2003**
(21) Application number: 00102057.7
(22) Date of filing: 02.02.2000
(51) Int. Cl.: G01N 33/68, C07K 14/47, C07K 16/18

(54) **Analytical method to evaluate non-human animal models of neurofibrillary degeneration**
Analytisches Verfahren zur Beurteilung von nicht-humanen Tiermodellen für neurofibrilläre Degenerationserkrankungen
Procédé analytique permettant l'évaluation de modèles animaux non-humains de la maladie d'Alzheimer

(43) Date of publication of application: 22.08.2001
(73) Proprietor: NADAG, 80339 München (DE)
(72) Inventor: Roder, Hanno, Dr., 80997 München (DE)
(74) Representative: Grund, Martin, Dr.

(56) References cited:
- GONG C X ET AL: "DEPHOSPHORYLATION OF ALZHEIMER'S DISEASE ABNORMALLY PHOSPHORYLATED TAU BY PROTEIN PHOSPHATASE-2A" NEUROSCIENCE,US,NEW YORK, NY, vol. 61, no. 4, 1994, pages 765-772, XP000578896 ISSN: 0306-4522
- DATABASE BIOSIS [Online] BIOSCIENCES INFORMATION SERVICE, PHILADELPHIA, PA, US; 1995 LEDESMA M DOLORES ET AL: "Isolation of a phosphorylated soluble tau fraction from Alzheimer's disease brain." Database accession no. PREV199598430679 XP002142567 & NEUROBIOLOGY OF AGING, vol. 16, no. 4, 1995, pages 515-522, ISSN: 0197-4580
- DATABASE MEDLINE [Online] US NATIONAL LIBRARY OF MEDICINE (NLM), BETHESDA, MD, US; IQBAL K ET AL: "Mechanism of neurofibrillary degeneration in Alzheimer's disease." retrieved from STN Database accession no. 95194583 XP002142568 & MOLECULAR NEUROBIOLOGY, (1994 AUG-DEC) 9 (1-3) 119-23. ,
- DATABASE BIOSIS [Online] BIOSCIENCES INFORMATION SERVICE, PHILADELPHIA, PA, US; 1993 SAUTIERE PIERRE-ERIC ET AL: "Alzheimer-type Tau epitopes detection after okadaic acid treatment of neuroblastoma cells." Database accession no. PREV199396066885 XP002142569 & COMPTES RENDUS DE L'ACADEMIE DES SCIENCES SERIE III SCIENCES DE LA VIE, vol. 316, no. 5, 1993, pages 533-535, ISSN: 0764-4469
- DATABASE BIOSIS [Online] BIOSCIENCES INFORMATION SERVICE, PHILADELPHIA, PA, US; 1996 HASEGAWA M ET AL: "Characterization of mAb AP422, a novel phosphorylation-dependent monoclonal antibody against tau protein." Database accession no. PREV199698785963 XP002142570 & FEBS LETTERS, vol. 384, no. 1, 1996, pages 25-30, ISSN: 0014-5793
- JOHNSON, GAIL V. W. ET AL: "Tau protein in normal and Alzheimer 's disease brain" ALZHEIMER'S DIS. REV. [ELECTRONIC PUBLICATION] (1996), 1(1/2), 38-54 , XP002142566

## Description

### Field of the Invention

The present invention relates to methods for modeling aspects of Alzheimer's disease (AD), in particular the present invention relates to methods for modeling abnormal tau hyperphosphorylation as the key step to the process of neurofibrillary degeneration and tau aggregation.

One of the major obstacles in the development of drugs for Alzheimer's disease (AD) is that there are no non-human animal models for all of the pathological aspects of the syndrome. The modeling of some aspects of the disease, like induction of amyloidosis in transgenic mice, has been demonstrated recently. However, in these models amyloidosis was found to not necessarily be associated with the clinically relevant process of neurodegeneration, as seen in AD, severely limiting the value of these models for drug development purposes.

For the process of neurofibrillary degeneration, possibly more relevant clinically, even selected aspects of this process have not been modeled. This may be due in part to the difficulty to assess in tissue specimen the biochemical state of the main constituent of neurofibrillary tangles, the microtubule-associated protein tau, which is often obscured by artifacts.

Cellular pathology as well as clinical symptoms are associated with aggregates of hyperphosphorylated tau, suggesting that this biochemical process is a crucial milestone to disease [Braak and Braak, Acta Neuropathol. 82, 239-259 (1991); Braak et al., Acta Neuropathol. 87, 554-567 (1994); Goedert et al., Neurobiol. Aging 16, 325-334 (1995)]. Unfortunately, despite many attempts, modeling of tau aggregation in conjunction with unambiguous hyperphosphorylation has proved to be elusive so far. In addition, it is very difficult to reliably assess the *normal* state of tau hyperphosphorylation in vivo. By definition, this uncertainty directly limits the definition of "abnormal" tauhyperphosphorylation. Thus it would be desirable to induce and assess authentic tau hyperphosphorylation in vivo in reference to normal phosphorylation, as a prerequisite to allow the assessment of the efficacy of drugs in preventing it.

### Background of the invention

A large number of antibodies has been developed in the past which either increase or decrease their reactivity with tau proteins after phosphorylation of specific subsets of phosphorylation sites or even individual sites (phosphorylation-dependent antibodies) [WO 93/112311]. Initially it was believed that a large number of these antibodies exhibit reactivity exclusively with pathologically modified tau in diseases like AD, where this form of the protein is associated with aggregates in dying neurons, hence the term "tau hyperphosphorylation" was used. However, it was soon recognized that this notion was based on an artifact. Many antibodies in fact reacted strongly with normal tau ex vivo when the time between death of the animal (or excision of human tissue in surgery) and extraction of tau was shortened to a few minutes [Matsuo et al., Neuron 13, 989-1002 (1994)]. Thus the artifact was explained by a rapid dephosphorylation of tau in post-mortem (or post-excision) tissue.

It was shown that abnormal tau is present in soluble non-PHF form in the Alzheimer's disease brain and the activities of phosphoseryl/phosphothreonyl protein 2A and non - receptor phosphotyrosyl phosphatase (s) are decreased in AD brain [Iqbal et al., Mol. Neurobiol. 9, 1994, pp. 119-123]. Furthermore, Ledesma et al., (Neuropbiology of Aging 16, 1995, pp. 515-522) showed that soluble tau species are present in Alzheimer but not in normal brain cytosol of post-mortem samples. Gong et al., (neuroscience 61/4, 1994, pp. 765-772) suggested a correlation between the activity of dephosphorylating activity of PP2A and the level of soluble Tau in the AD brain.

Nevertheless, some selected antibodies exhibited a substantially higher reactivity with pathological tau from AD brains than with rapidly extracted tau (a few minutes post-mortem or post-excision) from normal tissue, most notably the widely used reference mAb AT8 [Matsuo et al., Neuron 13, 989-1002 (1994)]. Although reactivity of AT8 with normal tau is considerably lower than with AD-tau, it still presents a formidable obstacle in the analysis of tau hyperphosphorylation in non-human animal models, as one is confronted with the task to differentiate the contribution of normal physiological processes to AT8 reactivity from the pathological contribution. Moreover, one cannot exclude the interpretation that any manipulation designed to model tau hyperphosphorylation in AD simply reduced the kinetics of post-mortem dephosphorylation as assessed with AT8, since the so-called "normal" AT8 reactivity might still be contaminated by a post-mortem artifact which occurs in the time frame of a few minutes. In the most extreme of interpretations, the seemingly hyperphosphorylated tau from AD brains might simply represent normally phosphorylated tau which is not subjected to the rapid post-mortem dephosphorylation in normal brains.

In spite of its limitations, AT8 is a sufficiently discriminating antibody to detect tau hyperphosphorylation induced by the protein phosphatase inhibitor okadaic acid in cell culture models, including freshly prepared brain slices (Example 4). Even the less discriminating antibodies PHF-1 and Tau-1 can still be used successfully in culture systems. This is due to the fact that in cellular systems AT8 reactivity of tau is either normally absent, or that the induction by okadaic acid affects a sufficiently large tau population to provide a clear difference to the normal level of AT8 reactivity. Moreover, there are no post-mortem artifacts in cellular systems. It could be reasonably expected that antibodies like AT8 might serve as well in the analysis of tau hyperphosphorylation *in vivo*.

So far tau phosphorylation in vivo has almost always been assessed by staining in tissue, as opposed to analysis of extracted tau. This is due to uncertainties concerning the existence in solution of hyperphosphorylated tau in AD brains because of analytical limitations. The hyperphosphorylated state is best demonstrated in association with the insoluble PHF (Paired 1-Helical Filament) aggregates [Lee et al., Science 251, 675-678 (1991); Hanger et al., Biochem. J. 275, 99-104 (1991)], because normally phosphorylated tau proteins are then absent, and the insoluble protein is more effectively protected from dephosphorylation artifacts than in the soluble state. Thus, non-human animal modeling experiments have focussed on the formation of aggregates in neurons as found in AD by tissue staining methods.

Single injection of okadaic acid into rat brain was reported to lead to immunohistochemical changes reminiscent of neurofibrillary changes shortly thereafter [Arendt et al., Neuroreport 5, 1397-1400 (1994)].

Sautière et al., (C.R. Acad. Sci. III 316, 1993, pp. 533-535) described an in vitro model of the inhibition of PP2A by okadaic acid and the production of abnormally phosphorylated Tau proteins. However, reactivity with a true phosphorylation-dependent antibody like AT8 was not demonstrated. The antibodies used (e.g. Alz-50) react even with unphosphorylated tau protein on Western-blots (Roder et al., Biochem. Biophys. Res. Commun. 193, 639-647 (1993)), i.e. the observed effect is possibly related to epitope exposure rather than phosphorylation. The discrepancy between staining on immunoblots and staining on tissue is common to virtually all known tau antibodies, and is explained by general masking of tau epitopes after fixation as used in histological tissue processing (e.g. Tashiro et al., Neuroreport 8, 2797-2801 (1997); Pollock and Wood, J. Histochem. Cytochem. 36, 1117- 1121 (1988)]. The observed "Alzheimer-like" changes can therefore not be interpreted unambiguously as pathological tau hyperphosphorylation, but could also be due to unmasking of normally present epitopes by the experimental manipulation.

Chronic infusion of okadaic acid into rat brain ventricles at the limit of toxicity for several weeks was again reported to result in histological staining reminiscent of AD, including staining with AT8 [Arendt et al., Neuroscience 69, 691-698 (1995)]. The analytical methods used again suffer from a series of limitations:
- Experiments have to be conducted for several weeks, which seriously limits usefulness for drug discovery and screening purposes
- Since okadaic acid has to be applied at the limit of toxicity, many animals die before any analysis can be conducted
- Because of long-term toxicity the results are often poorly reproducible (we have been completely unable to repeat any of the results)
- Any staining of tau in tissue is subject to artifactual epitope exposure and/or masking due to fixation effects: for instance aluminium intoxication has also been reported to result in AT8 tissue staining, however, this staining colocalized with staining by the antibody Tau- 1, clearly excluding an AD-like effect. The induction of AT8 (and Tau-1) reactivity under those conditions is probably related to exposure of normal epitope by unspecific toxicity, rather than biochemical tau hyperphosphorylation.
- It was claimed that on Western-blots tau proteins from normal rat brains were unreactive with AT8, while tau from okadaic acid treated rats was strongly reactive. The absence of normal AT8 reactivity of tau is in stark contrast to literature evidence [Matsuo et al., Neuron 13, 989-1002 (1994)1 and to data disclosed in this application; the likely explanation is that the normal reactivity was artifactually lost due to post-mortem dephosphorylation, and that the seemingly induced reactivity was rather a preservation of normal reactivity due to the presence of the phosphatase inhibitor okadaic acid.
- The former together with the observation described in this application of non-AD related induction of AT8 reactivity shows that the use of antibodies like AT8 for the analysis of tau hyperphosphorylation models is subject to serious misinterpretations.

At present non-human animal models of tau hyperphosphorylation cannot be reliably analyzed or even discovered. Post-mortem effects have usually not been excluded rigorously during characterization, e.g. [Jicha et al., J. Neurosci. 19, 7486-7494 (1999)], the epitopes of the used antibodies are often dependent on several phosphorylations or a combination of phosphorylation and protein folding, which would result from different biochemical pathways in vivo [WO 96/04309; Zheng-Fischhofer et al., Eur. J. Biochem. 252, 542-552 (1998); Jieha et al, J. Neurochem. 69, 2087-2095 (1997); Hoffmann et al., Biochemistry 36, 8114-8124 (1997)]. To model multiple pathways of AD in animals within a reasonable time frame is a tenuous endeavour depending on chance and therefore likely to be badly reproducible. For example, reactivity of tau against the mAb AT100 is already very difficult to reproduce in vitro [Zheng- Fischhofer et al., Eur. J. Biochem. 252, 542-552 (1998)]. None of the known methods have been shown to be useful for modeling aspects of Alzheimer's disease.

The foregoing illustrates that there is a need for a rapid, simple and reliable method for modeling aspects of Alzheimer's disease, that at the same time provides clarity of data interpretation free of artifacts and clearly separating normal from abnormal biochemistry in vivo, and free of the ethical constraints of long-term toxic treatment of unanesthesized animals.

### Summary of the Invention

The present invention relates to methods for modeling aspects of Alzheimer's disease (AD).

In one aspect of the invention there is provided a method for the determination of soluble hyperphosphorylated Tau in non-human animals, comprising: a) inhibition of protein phosphatase 2A (PP2A) in vivo; b) surgical removal of the brain or special regions of the brain; c) homogenization of the isolated tissue, ultracentrifugation or ultrafiltration and heat treatment of the supernatant, and d) assessing the presence of soluble hyperphosporylated tau.

The methods according to the present invention can be used e.g. for the determination of abnormal tau hyperphosphorylation as the key step to the process of neurofibrillary degeneration and tau aggregation.

The present invention provides for the first time a method for assessing the presence of soluble hyperphosporylated tau in non-human animal models.

In a preferred embodiment of the method according to the present invention step d) is performed with a primary polyclonal or monoclonal antibody raised against a peptide antigen containing the phosphorylated sequence Asp-(P)Ser-Pro with no other phophoamino acid present. In a particular preferred embodiment step d) is performed with an anti-phopho Ser422 antibody, and especially the antibody mAb AP422 [Hasegawa et al., FEBS Lett. 384, 25-30 (1996)].

An anti-phospho Ser422 (numbering according to the longest isoform of human tau) is an antibody which is directed to phosphoserine 422 of tau. For example, the antibody mAb AP422 has been characterized as a reagent to probe the phosphorylation state of Ser422 of tau. It is distinguished from other antibodies in that its epitope can only be produced by the kinase ERK2 and not by other kinases. However, its special utility for the characterization of non-human animal models vis-a-vis all other truly phosphorylation-dependent antibodies, of which AT8 was considered one of the best, has been unrecognized to date, because
(i) it was not expected to perform better in tissue staining than all other antibodies
(ii) its properties have been demonstrated only with insoluble PHF-tau from AD brains.

The invention also relates to method wherein the step of assessing the presence of soluble hyperphosporylated tau is performed by gentle fixation of soluble tau in tissue slices to avoid epitope masking followed by immunochemical analysis with an anti-phopho Ser422 antibody.

In a preferred embodiment the inhibition of protein phosphatase 2A (PP2A) in vivo is performed by direct injection or chronic infusion of okadaic acid into the brain of the animal.

In an alternative embodiment the inhibition of protein phosphatase 2A (PP2A) in vivo is performed by expression of transgenes suspected to be involved in the etiology of AD.

In a preferred embodiment step c) of the method according to the present invention comprises homogenization of the isolated tissue in ice-cold buffer of neutral pH containing 2µM okadaic acid and 2mM EDTA and 500 mM NaCl or other salts of equivalent ionic strength.

The invention provides a method for the unambiguous analysis in rodents of the characteristic hyperphosphorylation of soluble tau as associated with a variety of human neurodenerative conditions characterized and sometimes dominated by neurofibrillary degeneration, e.g. AD, frontal lobe dementia, Pick's disease, argyrophilic grains disease, and chromosome 17 dementias [Goedert et al.] in contrast to the more cumbersome, and up to now unsuccessful production of neurofibrillary degeneration. The invention comprises manipulations and the use of specific reagents to analyze the result of experiments designed to provoke tau hyperphosphorylation events in experimental animals within a short (days) timeframe.

The inventions shows:
- that there is more than one kind of tau hyperphosphorylation, and that antibodies directed to phosphoserine422 of tau differentiate the authentic process from tau hyperphosphorylation unrelated to disease, while the best prior art antibody AT8 does not.
- that tau hyperphosphorylation is associated with soluble tau species in human AD brain, instead of just the tau species forming the insoluble tangle; this shows that hyperphosphorylation of tau is a disease abnormality in its own right instead of just a by-product of tau aggregation, i.e. modeling of tau hyperphosphorylation is a relevant model for the disease.
- techniques to avoid artifactual alterations of the phosphorylation state ex vivo which routinely obscure the interpretation of in vivo processes.
- that antibodies directed against domains of tau protein which contain the Ser422 residue (numbering according to the longest human tau splice isoform) in a phosphorylated form are uniquely suited for the analysis of pathologically authentic tau hyperphosphorylation events in vivo.

The invention provides a solution to circumvent the modeling of hyperphosphorylated tau in the aggregates typical of AD by disclosing unexpected properties of a class of analytical tools which allow rapid and unambiguous assessment of pathological tau hyperphosphorylation in rodents. It centers around the insight that phosphorylation of the Ser422 residue in tau (numbering according to the longest isoform of human tau) is not only phosphorylated to a higher degree, as found for many phosphorylation domains, but is in fact qualitatively abnormal even in a soluble state of tau, i.e. there is no normal level of phosphorylation at this site at all. This provides a unique power of discrimination and clarity of interpretation which has previously not been recognized as mandatory for the analysis of non-human animal models.

### Description of the Drawings

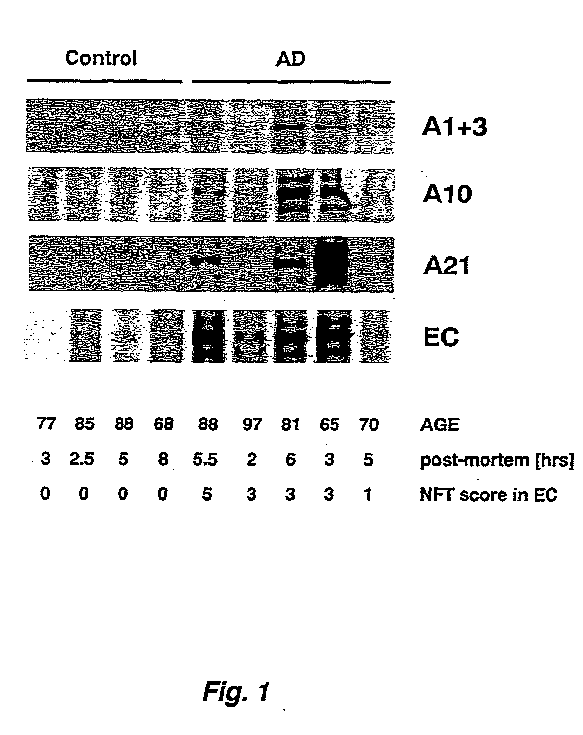

Fig. 1 shows immunoblots of heatstable supernatants of extracts from control and Alzheimer's disease brains. Upper Panels: Soluble heat-treated fractions from selected regions of human brain typically heavily affected by neurofibrillary degeneration in AD (A1+3, A10, A21: Broca brain areas; EC: entorhinal cortex) were probed with mAb AP422 for the presence of the typical PHF-tau protein "triplet" at the apparent molecular mass of 60-70 kD (shown here). Lower Panels: For each set of samples from individual patients the age, post-mortem time and the density of neurofibrillary tangles in the entorhinal cortex as assessed by Thioflavin S staining is given. Tangle density is reflected as a score from 1 = mild to 5 = severe according to the CERAD criteria. Note that the AD patient second from left was of extreme age and had the longest disease duration, probably reflecting a moderately aggressive course of the disease, while the AD patient on the far right with the lowest tangle score had a severe vascular amyloidosis, constituting a case of borderline diagnosis of AD.

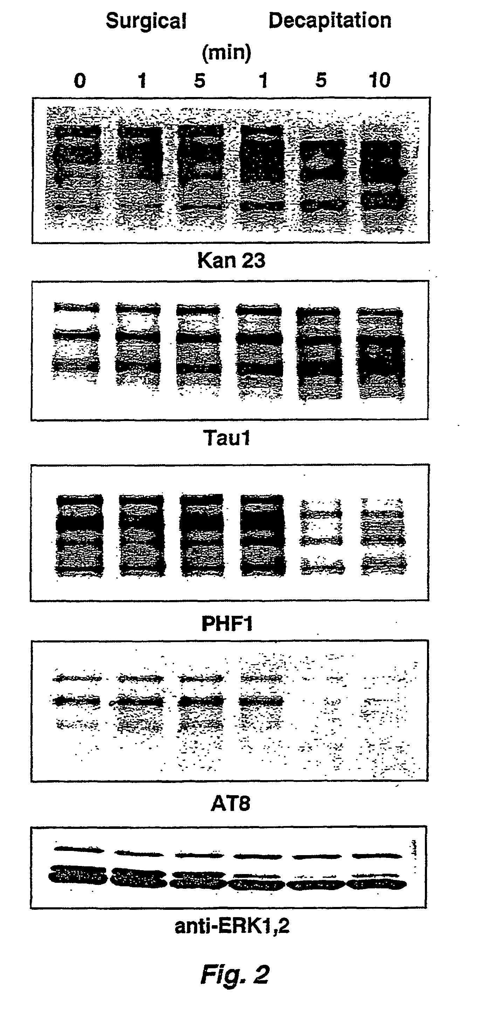

Fig. 2 shows a comparison of post-mortem dephosphorylation effects in rat brain with two different methods of brain recovery. 3 month old female Long-Evans rats were anethesized by i.p. injection of 65 mg/kg pentobarbital and subjected to either decapitation and resection of the brain (excluding cerebellum) as fast as possible (about I min) or surgical opening of the cranium with intact circulation and subsequent brain removal after transsection of the middle cerebral artery. Brains were quickly homogenized in ice-cold homogenization buffer 0-10 min post-resection. Supernatants after ultracentrifugation at 100,000 x g were boiled and aliquots of soluble fractions (about 3 µg protein each) were subjected to immunoblotting with a phosphorylation-independent polyclonal tau antibody, phosphorylation-dependent mAbs Tau-1, PHF-1, AT8, and a pan-ERK mAb from Zymed. Only the relevant parts of the immunoblots are shown (60-70 kD for tau, 40-45 kD for ERKs).

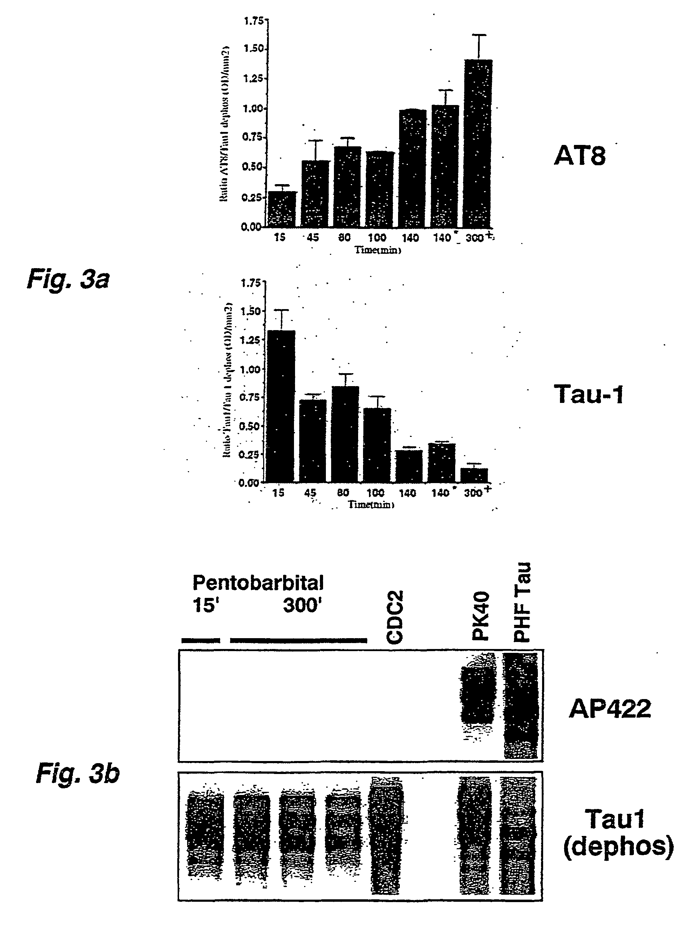

Fig. 3a shows AT8 and Tau-1 immunoreactivity changes under pentobarbital in rat brain. 3 month old female Long Evans rats were injected with 65 mg/kg Pentobarbital and subjected to surgical brain removal 15 min to 5 hrs later. To exclude that the observed tau immunochemical changes were due to a rapid dephosphorylation induced by pentobarbital followed by recovery of the normal state hours later a set of animals was injected a second time with 65 mg/kg pentobarbital after 2 hr 20 min. The animals at 5 hrs after the first anesthesia had to be anethesized a second time, as they had regained full consciousness. In no case was a lower state of phosphorylation observed after the second injection, indicating that the observed effect was due to increase of phosphorylation. Recovered brains were homogenized and supernatants processed for immunoblotting with AT8 and Tau-1 as before. To normalize signal intensity with phosphorylation-dependent mAbs to total amount of tau protein present in each extract (specific labelling) sister blots with a five-fold reduced load of extract protein were subjected to exhaustive dephosphorylation with calf intestinal alkaline phosphatase prior to staining with Tau-1. Blots were developed by ECL and relevant areas of the blots were scanned densitometrically. Ratios of signal intensities with AT8 or Tau-1 over intensities with Tau-1 after dephosphorylation are plotted; data represent triplicate experiments each.

Fig. 3b shows the analysis of rat brain tau phosphorylation states with mAb AP422. Extracts from surgically recovered rat brains which had been anesthesized briefly (15 min) and for a prolonged period of time (5 hrs; triplicate) were immunoblotted with mAb AP422. Equivalent amounts of normal rat brain tau were hyperphosphorylated exhaustively in vitro with either purified cdc2 or ERK2 kinase under comparable conditions, and were co-immunoblotted with the ex vivo tau samples and a comparable amount (see control staining with Tau-1 after dephosphorylation) of authentic PHF-tau from human AD brains as a reference.

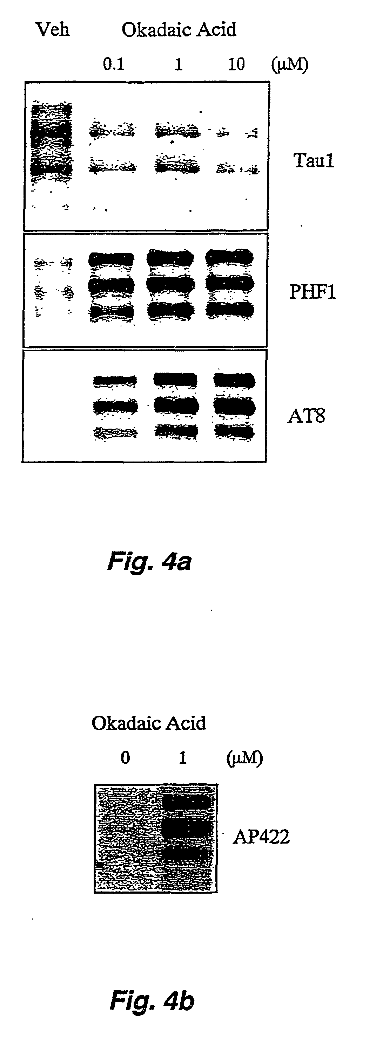

Fig. 4 shows the induction and analysis of tau hyperphosphorylation in rat brain slices. Freshly prepared hippocampal brain slices from adult Long-Evans rats were incubated under oxygenation in a physiological buffer for 1 hr with increasing concentrations of okadaic acid. Slices were homogenized in an ice-cold stop buffer by brief ultrasonication, and supernatants were boiled and analyzed for heatstable tau proteins by immunoblotting with reference mAbs Tau-1, AT8, and PHF-1 (Fig. 4a), and AP422 (Fig. 4b).

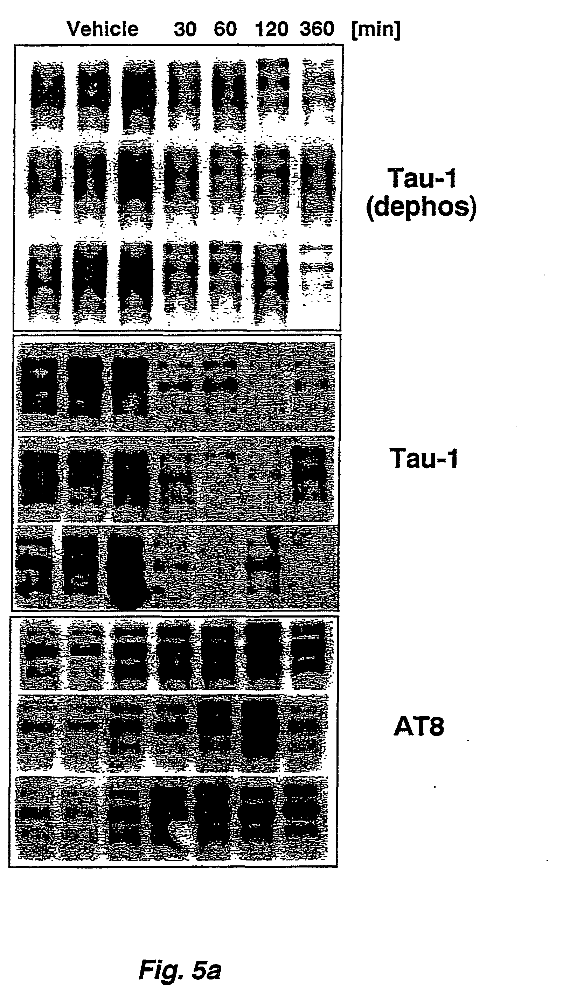

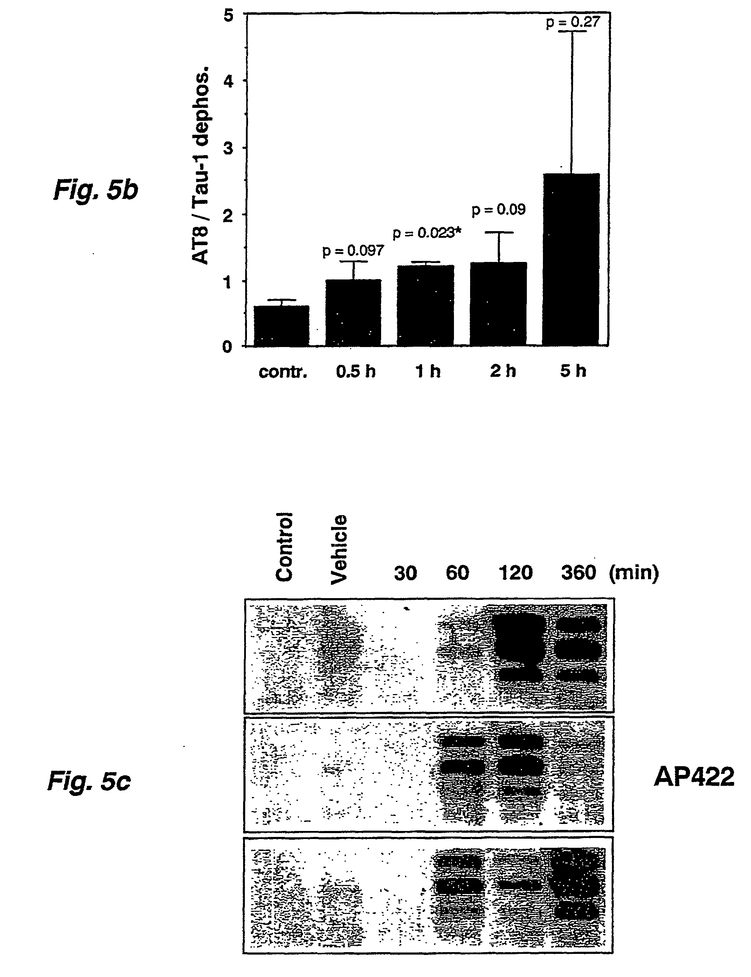

Fig. 5 shows the analysis of tau hyperphosphorylation in vivo with phosphorylation-dependent mAbs after intracerebral injection of okadaic acid.
a) Analysis with mAbs Tau-1 and AT8. 3 sets each of 3 month old female Long Evans rats were injected into the nucleus basalis with 3 µl vehicle in triplicate or with 3 µl 50 µM okadaic acid under pentobarbital anesthesia. Brains were surgically removed, the nucleus basalis dissected and homogenized in ice-cold stop buffer after 30 to 360 min. Heatstable supernatants were immunoblotted with Tau-1, AT8, and Tau-1 after dephosphorylation on the blot for normalization purposes.
b) Relevant areas of immunoblots developed with ECL were scanned densitometrically and AT8 signals normalized to Tau-1 signals of phosphatase-treated blots. Results represent triplicate experiments, P values were determined by ANOVA.
c) Aliquots of extracts of vehicle and okadaic acid injected nuclei basali were immunoblotted with mAb AP422.

### Description of specific embodiments

### Tau Hyperphosphorylation - A Model for Alzheimer's Disease

It is well established that the insoluble intracellular aggregates associated with the process of neurofibrillary degeneration in AD and a variety of similar diseases consist mainly of the microtubule-associated tau, and that this protein species is in a state of hyperphosphorylation. Less clear is, whether hyperphosphorylation is a by-product of aggregation, or whether it precedes aggregation as a causative event. In the latter case, it is expected to find soluble hyperphosphorylated tau in AD brains (i.e. not associated with tau aggregates). Since human AD brains are never available without a post-mortem delay of at least a few hours, the presence of tau reactivity with antibodies like AT8 in soluble brain extracts is never indicative of tau hyperphosphorylation, since this reactivity could always represent a disease-related lack of post-mortem dephosphorylation, sparing normal tau phosphorylation from removal. Again, the question of soluble tau hyperphosphorylation independent of aggregation cannot be addressed with antibodies like AT8, but requires an antibody like AP422 which has absolutely no reactivity against normal mammalian tau (see Example 3, Fig.3b). The lack of such analytical insights has limited efforts to establish non-human animal models on modeling of insoluble aggregates detectable by tissue staining.

It has been shown that hyperphosphorylated tau associated with the typical aggregates from AD brains is reactive with mAb AP422, as expected. However, the reactivity of soluble tau from AD brains has not been verified.

Indeed, tau proteins in human AD brain supernatants obtained after careful removal of any sedimentable material by ultracentrifugation is reactive with AP422, while reactivity in disease-free control samples from age-matched patients is essentially absent (Example 1, Fig.1; weak reactivity in the particularly susceptible entorhinal cortex may represent subclinical regeneration which is very frequent in old patients, or may represent AD in very early stages).

The demonstration of authentic hyperphosphorylated tau in a soluble state from AD brain justifies the utility of non-human animal models of tau hyperphosphorylation as models of an important disease process without the requirement of modeling the aggregation, which may require weeks or months, or may never occur with any tau species that is not human because of sequence differences.

### Detection and Analysis of Authentic AD-like Tau Hyperphosphorylation in Rat Brain

The usual procedure to obtain brains from rodents is to dissect the skull after decapitation under anesthesia (e.g. Pentobarbital). This procedure may take 2-3 minutes before the excised brain is ready for further processing. If the phosphorylation state of phosphoproteins of interest for AD pathology, namely tau and the kinase ERK2, is monitored by reactivity with several relevant phosphorylation-dependent antibodies, a uniform trend towards dephosphorylation is detected within minutes after the brain has been excised and has become accessible for analysis (Example 2, Fig.2). There is a decrease of reactivity with mAbs PHF- 1 and AT8, increase of reactivity with mAb Tau-1, and formation of tau species with a higher gel mobility on SDS-PAGE, all signs for tau dephosphorylation. The ERK2 phosphorylation state is monitored by gel mobility on SDS-PAGE and reactivity with a specific antibody directed at the doubly phosphorylated regulatory TEY motif of the kinase. Almost all of the ERK2 species is found in the high mobility form ex vivo, seemingly suggesting that the kinase Population is predominantly in the inactive state. Like with tau, further dephosphorylation/inactivation is seen in the minutes following excision.

With the above procedure it cannot be excluded that dephosphorylation is already proceeding during the few minutes needed to excise the brain, considering that the dephosphorylation events subject to monitoring occur in the time frame of minutes. Thus it is essential to eliminate the excision time.

Rats were anesthesized deeply with 65 mg/kg Pentobarbital for not longer than 10 minutes, before the skull was opened surgically, exposing the entire brain while maintaining full circulation. After transsection of the cerebral artery the brain was immediately homogenized into an ice-cold buffer containing phosphatase inhibitors and complexants of magnesium, effectively allowing to stop all phosphatase and kinase activities within 5s after excision. In this experimental paradigm, however, the tau as well as the ERK2 phosphorylation state was not appreciably altered even when the brain was homogenized up to 15 minutes after excision, indicating that the post-mortem dephosphorylation seen after decapitation did not occur at all when the brain was excised surgically from the live rat (Example 2, Fig.2). This procedure avoids the experimental dephosphorylation artifact and is therefore a necessary prerequisite for any valid analysis of normal or abnormal phosphorylation events in vivo.

The analysis of ERK2 phosphorylation/activation state demonstrates most clearly the importance to avoid brain excision artifacts- even with the most rapid dissection after decapitation it was impossible to capture ERK2 at any significant level of phosphorylation/activation. However, ex vivo analysis from surgically removed brains shows that constitutive phosphorylation and activation of almost half the ERK2 population in brain is a normal phenomenon, which is surprising considering that constitutive activation of this type of kinase has previously been described only in cancer cells.

Prolonged exposure of rats to pentobarbital beyond 15 minutes led to a show increase of AT8 reactivity and a correlated decrease of Tau-1 reactivity of tau, indicating a gradual increase of phosphorylation (Example 3, Fig.3a). AT8 reactivity normalized to total tau immunoreactivity had increased more than five-fold after five hours, approaching the level seen with PHF-associated tau isolated from AD brains. Rats had regained consciousness after this time and showed no abnormal autonomous signs. Judging by the criterion of AT8 and Tau-1 immunoreactivity as commonly accepted standards in the field, AD-like tau hyperphosphorylation had been induced in rat brain by prolonged pentobarbital treatment.

Only by using polyclonal or monoclonal antibodies raised against a peptide antigen containing the phosphorylated sequence Asp-(P)Ser-Pro with no other phosphoamino acid present, in particular the antibody mAb AP422 was it possible to clarify that the pentobarbital-induced tau hyperphosphorylation is completely unrelated to the tau phosphorylation in AD.

Unlike with AT8 as the best prior art mAb, reactivity of normal rat tau devoid of post-mortem dephosphorylation (excision time 5s, surgical brain removal) with AP422 is completely absent, and remains absent after pentobarbital-induced hyperphosphorylation of tau (Example 3, Fig.3b).

In contrast, PHF-tau from human AD brain is strongly reactive with AP422. The discrepancy is not explained by the species difference between tau from rats and humans, because full pathological AP422 reactivity of rat tau could be induced by phosphorylation of normal rat brain tau with purified ERK2 *in vitro*.

The unique discrimination power of anti-phosphoSer422 between different types of tau hyperphoshorylation is essential for a successful and convenient analysis of non-human animal models. The most straightforward means to induce tau hyperphosphorylation in biological model systems is the inhibition of PP2A, e.g. by okadaic acid. In cellular systems in vitro, e.g. brain slices, AT8 is sufficient to detect and monitor hyperphosphorylation of tau (Example 4, Fig.4a); AP422 reactivity is induced as well by okadaic acid (Fig.4b), satisfying the most discriminating criterion for a process related to AD pathophysiology. Because of reduced signal and increased variability in vivo, and modulation of underlying phosphorylation of tau by anesthetic methods, use of an antibody like AT8 for the analysis of phosphatase inhibitor-induced phosphorylation effects in brain is precluded. As there is no other known method of inducing authentic pathological tau hyperphosphorylation in biological systems, the analytical limitations are tantamount to the inability to pursue non-human animal models of tau hyperphosphorylation at all. Moreover, AT8 reactivity may be modulated simply by the amount of tau protein present in the sample: Thus, to allow comparison of phosphorylation status, the AT8 signal needs to be normalized to the total tau immunoreactivity as assessed by a phosphorylation-independent antibody or by immunoblotting after enzymatic removal of tau phosphorylation (e.g. treatment of blots with phosphatases). Inspection of AT8 immunoblots after okadaic acid injection in Fig.5a, and comparison of the normalized quantitated data in Fig.5b with Fig.3a clearly shows that any specific effect of okadaic acid is impossible to discern. In fact, variances are so dramatically increased so that hardly any useful significance is achieved. Thus, one cannot tell which individual animals or groups of animals had produced pathological tau hyperphosphorylation.

In contrast, when the same brain extract samples were analyzed with AP422, authentic AD-like tau hyperphosphorylation can be clearly detected in individual animals against virtually no background. The fact that *in vivo* tau hyperphosphorylation does not always occur in response to okadaic acid (Example 5, Fig.c) is surprising, as cellular systems, including brain slices, usually respond without exception. The causes for this variability in vivo are as yet unknown, but the clear-cut detection of this variation documents the discrimination power of antibodies directed against phosphoSer422, and shows that tau hyperphosphorylation is not a trival response *in vivo*.

Antibodies directed against phosphorylated Ser422 of tau are of superior utility, if not quintessential for the accurate analysis of non-human animal models of AD-related tau biochemistry.

The ability to assess this phenomenon in vivo with an method according to the present invention which can be performed within days as opposed to years in the case of transgenic animals is of great utility for a variety of purposes:
- Testing the efficacy and blood-brain barrier penetration of drugs inhibiting tau hyperphosphorylation, especially inhibitors of ERK2 and its regulators
- Determination of genetic background and susceptibility genes modulating the sensitivity of neurons to experience AD-like tau hyperphosphorylation
- Selection of experimental species and strains
- Identification of environmental factors modulating tau hyperphosphorylation
- Identification of humoral factors modulating tau hyperphosphorylation
- Identification and tracking of regulatory pathways and factors interfering with tau hyperphosphorylation

### Practice of the Invention

Small animals, e.g. rats and mice, are subjected to treatments designed to provoke hyperphosphorylation of tau in vivo. Such treatments may consist of direct or indirect methods. Direct methods may consist of direct injection or chronic infusion of pharmacological agents or toxins into the brain of the animal. Indirect methods may consist of the expression of transgenes suspected to be involved in the etiology of AD.

Analysis may be performed as follows:
Animals are anesthesized by agents commonly used in the art, including but not limited to barbiturates, ketamine, halothane, isoflurane. The skull is opened surgically, and the brain is removed after transsection of the middle cerebral artery. If desired, special regions of the brain may be dissected out within a few minutes. The isolated tissue is then homogenized in a small volume of ice-cold buffer of neutral pH containing 2 µM okadaic acid and 2mM EDTA to block kinases and phosphatases, protease inhibitors to block proteolytic activities, and 500 mM NaCl or other salts of equivalent ionic strength. The homogenate is subjected to centrifugation at 100,000 x g for 30 min, and the supernatant is boiled for 10 min. Coagulated protein is removed by centrifugation at 16,000 x g, and the supernatant containing heat-stable proteins is dialysed into a neutral low ionic strength buffer. Samples are concentrated by ultrafiltration as needed for SDS-PAGE analysis and immunoblotting. The phosphorylation state of tau proteins on immunoblots is assessed by a primary polyclonal or monoclonal antibody raised against a peptidic antigen containing the phosphorylated sequence Asp- (P)Ser-Pro with no other phosphoamino acid present. The antibody may be labelled directly with biotin, digitoxigenin, peroxidase, phosphatase, radioisotopes, or any other method commonly used in the art, or by a secondary antibody laballed in the same fashion. Equivalent methods to assess the presence of soluble hyperphosphorylated tau may be used, e.g. gentle fixation of soluble tau in tissue slices to avoid epitope masking followed by immunochemical analysis with an anti-phosphoSer422 antibody. Fixation of soluble tau species may be performed by brief exposure to ice-cold mixtures of paraformaldehyde/glutaraldehyde in phosphate buffers [Dotti et al., Neuroscience 23, 121-130 (1987)], Periodate/lysine/paraformaldehyde [Pollock and Wood, J. Histochem. Cytochem. 36, 1117-1121 (1988)] or similar mildly acting fixatives.

The present invention will now be illustrated by the following examples, which are not intended to be limiting in any way.

### Examples

### Example 1

Small tissue samples from human autopsy brain with or without AD as confirmed by neuropathologieal examination were powderized at -78°C and homogenized in 2 ml of ice- cold homogenization buffer 0 by sonication. Insoluble material was removed by centrifugation for 30 min at 120,000 x g and supernatants were subjected to boiling for 5 min followed by centrifugation at 13,000 x g for 5 min to yield heat stable supernatants. Samples were dialyzed into a low salt buffer (10 mM BisTris, pH 7.0, 1 mM EDTA).

Aliquots of samples from Brodman areas Al+3, A10, A21 and the entorhinal cortex corresponding to about 15 µg protein were separated on 10% SDS-PAGE (Novex) and immunoblotted on nitrocellulose. Membranes were blocked for 1 hr with 3% BSA (Sigma, immunoglobulin-free grade), 10 mM PBS, pH 7.2 (blocking buffer). Blots were incubated overnight with AP422 in 10 mM PBS, pH 7.2, 0.5% Triton X100, 2% normal goat serum (5% for secondary antibody), washed several times with the same buffer, and developed using ECL (enhanced chemiluminescence) Western-blotting protocol (Amersham Life Science) with horseradish peroxidase-linked sheep anti-mouse secondary antibody (1 : 3,000).

### Example 2

3 month old female Long-Evans rats were anesthesized with 65 mg/kg pentobarbital i.p. After 10-15 min rats were decapitated and the brain dissected as rapidly as possible (about 1 min) or with a further delay of up to 5 min. After removal of the cerebellum brains were processed as described below.
Alternatively, skulls of anesthesized rats were opened surgically with maintenance of circulation, and brains were immediately removed after transsection of the spinal cord (see example 5). After removal of the cerebellum cortices were processed either immediately or after having been left at ambient temperature for time delays of up to 10 min.

Brains were processed by homogenization in 4 ml ice-cold homogenization buffer (100 mM KH₂PO₄, pH 6.5, 2 mM EGTA, 2 mM EDTA, 0.5 mM PMSF, 2 µM okadaic acid, and 10 µg/ml leupeptin) with an Ultra-Turax followed by centrifugation at 16,000 x g for 30 min at 4°C. Aliquots were removed and boiled with an equal volume of Laemmli SDS sample buffer for ERK immunoblotting. The remainder of the supernatants was boiled for 5 min and insoluble material was removed by centrifugation at 16,000 x g for 30 Min. Heat-stable supernatants were dialyzed into a low salt buffer (10 mM BisTris, pH 7.0, 1 mM FDTA) and aliquots containing 15 µg protein were separated by 10% SDS-PAGE followed by immunoblotting overnight at 4°C on nitrocellulose. Blots were blocked with blocking buffer for 1 hr, washed, and incubated for at least 4 hrs with the following antibodies in 10 mM PBS, pH 7.2, 0.5% Triton X100, 1 % BSA.- phosphorylation-independent pAb anti-tau256-273 (1 : 1,000), Tau-1 (Boehringer Mannheim) at 1 : 5,000, PHF-1 at 1 : 1,000, and AT8 (Biosource International) at 1 : 200.

For ERK blotting samples were analyzed on 12% SDS-PAGE (Novex) and transferred to nitrocellulose. After blocking blots were incubated with anti-ERK mAb Z033 (Zymed) at 1 : 5,000. After repeated washing in 10 mM PBS, pH 7.2, 0.5% Triton X100, primary antibodies were detected after incubation overnight with alkaline-phosphatase coupled goat (for rabbit primary pAbs) or rabbit (for mouse primary mAbs) at 1 - 3,000 by a nitro blue tetrazolium staining kit (Life Technologies).

### Example 3

3 month old female Long Evans rats were left for 15 to 300 min after anesthesia with 65 mg/kg pentobarbital i.p. before surgical removal of the brain. A second dose of anesthetic was administered immediately prior to brain removal when excision occurred two hours or more after the first anesthesia. Cortices were processed and tau proteins analyzed by Western-blotting as in Example 2, using mAbs Tau-1 and AT8. To allow quantitative immunochemical evaluation of tau phosphorylation sister blots of all samples with a five-fold lower protein load were subjected to exhaustive dephosphorylation to provide a relative measure for total load of tau protein using Tau-1 staining. Blots blocked with BSA were incubated for 16 hrs at 37°C with 100 U/ml alkaline phosphatase (Gibco BRL) in 5 ml of 50 mM TBS, pH 8.5, 0.1 mM EDTA. All blots were developed using an ECL protocol with HRP-linked secondary sheep anti-mouse pAb. To ensure comparability blots from a series of experiments were strictly codeveloped for each antibody in the same process. Signals were captured on KODAK X-OMAT scientific imaging film and quantitated by densitometric scanning and image analysis with NIH image 1.44. Levels of phosphorylation were plotted after forming the ratio of phosphorylation-dependent immunosignals with Tau-1 or AT8 over phosphorylation-independent signals with Tau-1 after stripping blotted tau proteins enzymatically of phosphates to normalize signals for the amount of tau proteins present (Fig. 3a).
To compare the performance of AT8 vs. AP422 and to interpret the tau phosphorylation events more accurately, selected aliquots of samples analyzed with AT8/Tau-1 were also analyzed by immunoblotting with AP422 as described in Example 1. For reference purposes comparable amounts of normal rat tau proteins (after 15 min pentobarbital anesthesia) were incubated overnight with excess cdc2 kinase and the ERK2 kinase PK40 from bovine brain in 50 mM HEPES, pH 7.0, 2 mM Mg²⁺, 1 mM ATP, 1 mM DTT at 37°C. Kinase reactions were stopped by SDS-PAGE sample buffer prior to analysis on SDS-PAGE. As a further reference for authentic human AD-tau partially resolubilized hyperphosphorylated tau fractions from a preparation of sarcosyl-insoluble PHF-tau was loaded alongside on the same immunoblot (Fig. 3b).

### Example 4

Adult male Long-Evans rats were anesthesized with CO₂ and decapitated. Brains were removed within 2 min and the hippocampus was dissected using a blunt spatula. Hippocampi were cut into 0.45 mM slices using a Mcllwain tissue chopper and placed into ice-cold low Ca²⁺ Krebs-Bicarbonate buffer (pH 7.0): 124 mM NaCl, 3.33 mM KCl, 0.01 mM Ca²⁺, 1.25 mM KH₂PO₄, 1.33 mM MgSO₄, 25.7 mM NaHCO₃, 10 mM D-Glucose, 20 mM HEPES. 5-8 slices were placed into a tube with 5 ml of low Ca²⁺ buffer and incubated for at least 30 min at 33-34°C with water saturated oxygenation (95% O₂, 5% CO₂.). After 30 min the solution was replaced with a buffer containing a physiological level of Ca²⁺ (1.3 mM) and incubated for an additional 30 min. After a total equilibration period of at least 1 hr, the slices were exposed for 90 min to increasing concentrations of okadaic acid up to 10 mM. After this treatment the buffer was removed and the slices sonicated for 10-20 s in 0.5 ml homogenization buffer (see example 2) containing a cocktail of protease inhibitors: 100 µM PMSF, 10 µg/ml aprotinin, 10 µM leupeptin, 6 µg/ml pepstatin, 40 µM chymostatin. Homogenates were centrifuged for 30 min at 16,000 x g, supernatants were collected, heated for 5 min to 100°C and centrifuged again. Aliquots of heat-stable supematants, normalized for protein content, were analyzed on 10% SDS-PAGE, followed by immunoblotting with mAbs AT8 (1:200) and AP422 (1 : 5,000). Blots were developed by ECL (Amersham Life Sciences) and exposed to a Kodak X-OMAT AR film.

### Example 5

Male or female Long-Evans rats at approximately 3 to 4 months of age were housed in pairs with food and water available ad libidum. The animals were anesthesized deeply with pentobarbital (65 mg/kg, i.p.) followed by intracerebral injection of okadaic acid: A small incision was made in the scalpe and the skull was exposed. The tissue was held out of the operating field with forceps and the areas of injection were marked. Openings were made through the skull using a dentists drill and a number 5 carbide bur. Micro injections of 2.5 µl of a 100 µM of okadaic acid in vehicle (10 mM PBS, pH 7.2) were made into the basal nucleus (location from bregma -0.18 mm AP, +/-0.30 mm midline, and from the dura- 0.69 mm) using a 10 µl Hamilton syringe mounted in a micro injection unit (model 5000; David Kopf Instruments). The infusion time for the 2.5 µl of fluid was approximately 10 minutes. 30 to 360 min after the okadaic acid injection rats were subjected to the surgical brain removal procedure. When surgery was performed more than 2 hrs after the first dose of anesthesia, a second injection of pentobarbital was given i.p. Each experimental condition was performed on a set of three animals. Controls included injection of equivalent amount of vehicle, or no injection at all.
Surgery was performed in a Kopf stereotaxic apparatus (model 900; David Kopf Instruments, Tajumga, CA). An enlarged incision was made in the scalp and the skin was pushed to the sides and held out of the operating field with forceps. Using a dentist's drill with a micro dissecting trephine attachment (10 mm), the skull was removed to allow free access to the brain. The brain was severed from the spinal cord followed by dissection of the nuclei basali as 3x3 mm tissue blocks which were immediately homogenized in 0.5 ml ice-cold homogenization buffer by sonication (see example 2). Extracts were centrifuged and heat- treated as described in example 4.
Heat-stable supernatants of triplicate experiments were analyzed by imrnunoblotting with AT8 (Fig.5a) and AP422 (Fig.5c). AT8 signals were quantitated after normalization to Tau-1 immunoreactivity on phosphatase-treated blots as described in example 3. Means of such ratios (n = 3) were tested for significance by a standard ANOVA analysis (Fig.5b).

## Claims

1. Method for the determination of soluble hyperphosphorylated tau in non-human animals, comprising
a) inhibition of protein phosphatase 2A (PP2A) in vivo
b) surgical removal of the brain or special regions of the brain
c) homogenization of the isolated tissue, ultracentrifugation or ultrafiltration and heat-shock treatment of the supernatant,
d) assessing the presence of soluble hyperphosporylated tau.

2. Method according to claim 1 wherein step d) is performed with a primary polyclonal or monoclonal antibody raised against a peptide antigen containing the phosphorylated sequence Asp-(P)Ser-Pro with no other phosphoamino acid present.

3. Method according to claim 1 or 2 wherein step d) is performed with an anti-phosphoSer422 antibody.

4. Method according to claim 3, wherein the anti-phosphoSer422 antibody is monoclonal antibody AP422.

5. Method according to claim 4 wherein step d) is performed by gentle fixation of soluble tau in tissue slices to avoid epitope masking followed by immunochemical analysis with an anti-phosphoSer422 antibody.

6. Method according to any one of claims 1 to 5 wherein step a) is performed by direct injection or chronic infusion of pharmacological agents or toxins into the brain of the animal.

7. Method according to claim 6, wherein step a) is performed by direct injection or chronic infusion of okadaic acid into the brain of the animal.

8. Method according to any one of claims 1 to 5 wherein step a) is performed by expression of transgenes suspected to be involved in the etiology of AD.

9. Method according to any one of claims 1 to 8 wherein step c) comprises homogenization of the isolated tissue in ice-cold buffer of neutral pH containing 2µM okadaic acid and 2mM EDTA and 500 mM NaCl or other salts of equivalent ionic strength.

10. Method according to claim 9 wherein step c) further comprises centrifugation at >100000 x g for about 15-30 min or ultrafiltradon with a cut-off of 100 kD and boiling of the supernatant for about 5-10 min.

11. Method according to any one of claims 1 to 10 for the determination of abnormal tau hyperphosphorylation as the key step to the process of neurofibrillary degeneration, and tau aggregation.

## Patentansprüche

1. Verfahren zur Bestimmung von löslichem, hyperphosphoryliertem Tau in nichthumanen Tieren, umfassend
a) Inhibition der Protein-Phosphatase 2A (PP2A) in vivo
b) chirurgisches Entfemen des Gehirns oder bestimmter Regionen des Gehirns
c) Homogenisierung des isolierten Gewebes, Ultrazentrifugation oder Ultrafiltration und Hitzeschock-Behandlung des Überstandes
d) Bestimmen der Anwesenheit von löslichem, hyperphosphoryliertem Tau.

2. Verfahren gemäß Anspruch 1, wobei Schritt d) mit einem primären polyklonalen oder monoklonalen Antikörper durchgeführt wird, der gegen ein Peptidantigen gebildet wurde, das die phosphorylierte Sequenz Asp-(P)Ser-Pro mit keiner anderen anwesenden Phospo-Aminosäure enthält.

3. Verfahren gemäß Anspruch 1 oder 2, wobei Schritt d) mit einem Anti-PhosphoSer422 Antikörper durchgeführt wird.

4. Verfahren gemäß Anspruch 3, wobei der Anti-PhosphoSer422 Antikörper der monoklonale Antikörper AP422 ist.

5. Verfahren gemäß Anspruch 4, wobei Schritt d) ausgeführt wird durch vorsichtiges Fixieren von löslichem Tau in Gewebeschnitten, um Epitop-Maskierung gefolgt von immonochemischer Analyse mit einem Anti-PhospoSer422 Antikörper zu vermeiden.

6. Verfahren gemäß einem der Ansprüche 1 bis 5, wobei Schritt a) ausgeführt wird durch direkte Injektion oder chronische Infusion von pharmakologischen Mitteln oder Toxinen in das Gehirn des Tieres.

7. Verfahren gemäß Anspruch 6, wobei Schritt a) ausgeführt wird durch direkte Injektion oder chronische Infusion von Okadainsäure in das Gehim des Tieres.

8. Verfahren gemäß einem der Ansprüche 1-5, wobei Schritt a) ausgeführt wird durch Expression eines Transgens, von dem vermutet wird, dass es in die Ätiologie von AD involviert ist.

9. Verfahren gemäß einem der Ansprüche 1 bis 8, wobei Schritt c) die Homogenisierung des isolierten Gewebes in eiskaltem Puffer mit neutralem pH, der 2 µM Okadainsäure und 2 mM EDTA und 500 mM NaCI oder andere Salze entsprechender lonenstärke enthält, umfasst.

10. Verfahren gemäß Anspruch 9, wobei Schritt c) weiterhin die Zentrifugation bei >100000 x g für etwa 15-30 Min oder die Ultrafiltration mit einem *Cut-off* von 100 kD und Kochen des Überstandes für etwa 5-10 Min umfasst.

11. Verfahren gemäß einem der Ansprüche 1 bis 10 zur Bestimmung abnormaler Tau-Hyperphosphorylierung als ein Schlüsselschritt in den Prozess der neurofibrilären Degeneration und Tau Aggregation.

## Revendications

1. Procédé de détermination de tau soluble hyperphosphorylé chez des animaux non-humains, comprenant
(a) le fait d'inhiber la protéine phosphatase 2A (PP2A) *in vivo*
(b) l'ablation chirurgicale du cerveau ou de régions spéciales du cerveau
(c) l'homogénéisation du tissu isolé, l'ultracentrifugation ou l'ultrafiltration et un traitement de choc à la chaleur du surnageant,
(d) le fait de tester la présence de tau soluble hyperphosphorylé.

2. Procédé selon la revendication 1 dans lequel l'étape (d) est effectuée avec un anticorps primaire polyclonal ou monoclonal dirigé contre un antigène peptidique contenant la séquence phosphorylée Asp-(P)Ser-Pro avec aucun autre acide phosphoaminé présent.

3. Procédé selon la revendication 1 ou 2, dans lequel l'étape (d) est effectuée avec un anticorps antiphosphoSer422.

4. Procédé selon la revendication 3, dans lequel l'anticorps antiphosphoSer422 est un anticorps monoclonal AP422.

5. Procédé selon la revendication 4, dans lequel l'étape (d) est effectuée par une fixation douce de tau soluble sur des coupes de tissu pour éviter le masquage de l'épitope suivi par une analyse immunochimique avec un anticorps antiphosphoSer422.

6. Procédé selon l'une quelconque des revendications 1 à 5, dans lequel l'étape (a) est effectuée par une injection directe ou une perfusion chronique d'agents pharmacologiques ou de toxines dans le cerveau de l'animal.

7. Procédé selon la revendication 6, dans lequel l'étape (a) est effectuée par une injection directe ou une perfusion chronique d'acide okadaïque dans le cerveau de l'animal.

8. Procédé selon l'une quelconque des revendications 1 à 5, dans lequel l'étape (a) est effectuée par l'expression de transgènes potentiellement impliqués dans l'étiologie de AD.

9. Procédé selon l'une quelconque des revendications 1 à 8, dans lequel l'étape (c) comprend l'homogénéisation du tissu isolé dans un tampon froid à pH neutre contenant 2 µM d'acide okadaïque et 2 mM d'EDTA et 500 mM NaCl ou d'autres sels de force ionique équivalente.

10. Procédé selon la revendication 9, dans lequel l'étape (c) comprend de plus une centrifugation à une vitesse supérieure à 100 000 x g pendant environ 15 à 30 minutes ou une ultracentrifugation avec une limite de 100 kD et l'action de faire bouillir le surnageant pendant à peu près 5 à 10 minutes.

11. Procédé selon l'une quelconque des revendications 1 à 10 pour la détermination d'une hyperphosphorylation anormale de tau en tant qu'étape clé du procédé de dégénération neurofibrillaire et d'agrégation de tau.
